# NEUE EUROPÄISCHE PATENTSCHRIFT

(11) **EP 3 082 959 B2**
(45) Veröffentlichungstag und Bekanntmachung des Hinweises auf die Entscheidung über den Einspruch: **04.12.2024**
(45) Hinweis auf die Patenterteilung: 04.09.2019
(21) Anmeldenummer: 14798825.7
(22) Anmeldetag: 13.11.2014
(51) Int. Cl.: A61Q 5/02, A61Q 5/12, A61K 8/73, A61K 8/891, A61K 8/92, A61K 8/65

(54) **KOSMETISCHES HAARREINIGUNGSMITTEL**
COSMETIC HAIR CLEANSING COMPOSITION
COMPOSITION COSMÉTIQUE À PURIFIER LES CHEVEUX

(30) Priorität: 17.12.2013 DE 102013226269
(43) Veröffentlichungstag der Anmeldung: 26.10.2016
(73) Patentinhaber: Henkel AG & Co. KGaA, 40589 Düsseldorf (DE)
(72) Erfinder: SCHRÖDER, Thomas, 22395 Hamburg (DE); HENTRICH, Dirk, 22457 Hamburg (DE); BENDER, Corinna, 22457 Hamburg (DE)
(74) Vertreter: LKGLOBAL Lorenz & Kopf Patentanwalt Attorney at Law PartG mbB
(86) Internationale Anmeldenummer: PCT/EP2014/074436
(87) Internationale Veröffentlichungsnummer: WO 2015/090743

(56) Entgegenhaltungen:
- WO-A2-2013/117464
- CN-A- 102 475 639
- ANONYMOUS: "Sonnenschein 86 Blog", ULTIMATE OIL ELIXIR, 22 March 2013 (2013-03-22), pages 1 - 7
- ANONYMOUS: "Shampoo 2029093", MINTEL, 1 March 2013 (2013-03-01), pages 1 - 2
- ANONYMOUS: "Shampoo 2084214", MINTEL, 1 June 2013 (2013-06-01), pages 1 - 2
- ANONYMOUS: "Shampoo 2051222", MINTEL, 1 April 2013 (2013-04-01), pages 1 - 2

## Beschreibung

Die Erfindung liegt auf dem Gebiet der Kosmetik und betrifft Reinigungsmittel, die ein anionisches und/oder amphoteres Tensid, ein kationisches Polysaccharid, eine Silikon, ein Wachs, ein natives Öl und ein kationisches Proteinhydrolysat enthalten.

Die Erfindung betrifft weiterhin die Verwendung der Reinigungsmittel zur Beseitigung, Verminderung und/oder Vermeidung von Haarbruch sowie ein Verfahren zur Reinigung von Haaren und/oder zur Beseitigung, Verminderung und/oder Vermeidung von Haarbruch, bei dem ein erfindungsgemäßes Reinigungsmittel auf die Haare aufgebracht, und nach einer Einwirkungszeit wieder ausgespült wird.

Kosmetische Reinigungsmittel, wie beispielsweise Haarshampoos, basieren auf klassischen anionischen, amphoteren, zwitterionischen, nichtionischen und/oder kationischen Tensiden.

Aufgrund ihres hervorragenden Reinigungs- und Schaumvermögens werden überwiegend anionische Tenside, gegebenenfalls in der Mischung mit geringen Mengen an Co-Tensiden, eingesetzt.

Ein solches handelsübliches Shampoo reinigt die Haare und beseitigt Talg- und/oder Rückstände von Stylingmitteln sowie andere Verschmutzungen von der Haaroberfläche und der Kopfhaut.

Während der Reinigung werden aus den Haaren und der Kopfhaut aber auch Lipide und Proteine entfernt, wodurch insbesondere bei häufiger Reinigung eine Schädigung der Haarstruktur und/oder ein Austrocknen der Kopfhaut auftreten kann.

Schädigungen der Haarstruktur oder der Haarfasern, insbesondere Spliss und/oder Haarbruch, können zudem durch Umwelteinflüsse (wie beispielsweise intensive Sonneneinstrahlung), mechanische Belastungen (wie beispielsweise kämmen unter Föhnhitze) sowie durch chemische Einflüsse (wie beispielsweise Färben, Verformen oder Glätten der Haare) begünstigt werden.

Aus dem Stand der Technik sind zahlreiche Haarpflegemittel wie Haarkuren, Spülungen oder Haarpflegelotionen bekannt, die erfolgreich für die Behandlung von Spliss und/oder Haarbruch angewendet werden können.

Für den täglichen Gebrauch werden von den Verbrauchern aber aus Zeit- und Kostengründen Haarreinigungsmittel gewünscht, die gleichzeitig einen Pflege- und Reparatureffekt auf den Haaren bewirken.

Die Formulierung so genannter 2in1- oder 3in1-Präparate gehört ebenfalls seit längerer Zeit zum Stand der Technik. So wird in DE 102012201861 ein konditionierendes Haarreinigungsmittel vorgeschlagen, das neben einem anionischen Tensid ein kationisches Guar-Polymer, eine Silikonemulsion und mindestens ein Wachs enthält. Die Reinigungsmittel sind schaumstark und verleihen den Haaren nach ihrer Anwendung eine verbesserte Kämmbarkeit sowie verbesserte optische und haptische Eigenschaften.

Es wurde jedoch gefunden, dass diese Mittel sowie vergleichbare 2in1-Reinigungspräparate, die aus dem Stand der Technik bekannt sind, insbesondere im Hinblick auf die Reparatur geschädigter Haarfasern sowie auf die Kräftigung der Haarstruktur weiter optimiert werden müssen.

Der vorliegenden Anmeldung lag die Aufgabe zugrunde, milde, haut- und haarverträgliche, pflegende Haarreinigungsmittel mit guter Wirksamkeit gegen Haarschädigungen bereitzustellen, die optimalerweise die Haarstruktur kräftigen.

Es wurde nun gefunden, dass diese Aufgabe durch eine spezifische Mischung aus mindestens einem Tensid und speziellen Pflegestoffen gelöst werden kann. Die entsprechenden Reinigungsmittel sind sehr gut haut- und haarverträglich, sie pflegen und reinigen das Haar, kräftigen die Haarstruktur und vermindern signifikant Haarschädigungen, insbesondere Haarbruch.

Ein erster Gegenstand der Erfindung ist daher ein kosmetisches Reinigungsmittel, das in einem kosmetischen Träger
a) mindestens ein anionisches und/oder amphoteres (zwitterionisches) Tensid,
b) mindestens ein kationisches Polysaccharid,
c) mindestens eine Silikon, das ausgewählt ist aus wasserunlöslichen, nicht-flüchtigen Polydimethylsiloxanen, die bei 25°C eine Viskosität im Bereich von 30.000 bis 100.000 cSt aufweisen,
d) mindestens ein Wachs,
e) mindestens ein natives Öl und
f) mindestens ein kationisches Proteinhydrolysat enthält, wobei der Gewichtsanteil des kationischen Proteinhydrolysats am Gesamtgewicht der Zusammensetzung 0,01 bis 3 Gew.-% beträgt, wobei der Gewichtsanteil des nativen Öls am Gesamtgewicht der Zusammensetzung 0,01 bis 3 Gew.-% beträgt.

Unter einem kosmetisch akzeptablen Träger wird im Rahmen der Erfindung bevorzugt ein wässriger oder wässrig-alkoholischer Träger verstanden. Besonders bevorzugt enthält der kosmetische Träger mindestens 50 Gew.-%, mehr bevorzugt mindestens 60 Gew.-% und besonders bevorzugt mindestens 65 Gew.-% Wasser.

Weiterhin kann der kosmetische Träger 0,01 bis 40 Gew.-%, bevorzugt 0,05 bis 30 Gew.-% und insbesondere 0,1 bis 20 Gew.-% mindestens eines Alkohols enthalten, der ausgewählt sein kann aus Ethanol, Ethyldiglykol, 1-Propanol, 2-Propanol, Isopropanol, 1,2-Propylenglycol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycolen, Sorbitol, Sorbitan, Benzylalkohol, Phenoxyethanol oder Mischungen dieser Alkohole.

Besonders bevorzugt sind die wasserlöslichen Alkohole.

Insbesondere bevorzugt sind Ethanol, Ethyldiglykol, 1-Propanol, 2-Propanol, Isopropanol, 1,2-Propylenglycol, Glycerin, Benzylalkohol und/oder Phenoxyethanol sowie Mischungen dieser Alkohole.

Zu den geeigneten anionischen Tensiden, die in den erfindungsgemäßen Reinigungsmitteln eingesetzt werden können, zählen beispielsweise:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfate und/oder Alkylpolyglykolethersulfatsalze der Formel R-O-(CH₂-CH₂O)ₙ-CH₂-CH₂-O-SO₃X, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen, und/oder
- Alkyl- und/oder Alkenyletherphosphate der Formel 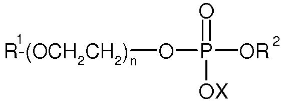 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder die Gruppe -NR³R⁴R⁵R⁶ steht, mit R³ bis R⁶ unabhängig voneinander stehend für einen C₁ bis C₄ -Kohlenwasserstoffrest.

Bevorzugte anionische Tenside sind Alkylsulfate und/oder Alkylpolyglykolethersulfatsalze der Formel R-O-(CH₂-CH₂O)ₙ-CH₂-CH₂-O-SO₃X, in der R bevorzugt für einen geradkettigen oder verzweigten, gesättigten oder ein- bzw. mehrfach ungesättigten Alkyl- oder Alkenlyrest mit 8 bis 24 Kohlenstoffatomen, n für 0 oder 1 bis 12 und X für ein Alkali- oder Erdalkalimetall oder für Triethanolamin steht.
Besonders bevorzugte anionische Tenside sind geradkettige oder verzweigte Alkylpolyglykolethersulfatsalze der zuvor genannten Formel, die einen Alkylrest mit 8 bis 18 und insbesondere mit 10 bis 16 C-Atomen sowie 1 bis 6 und insbesondere 2 bis 4 Ethylenoxideinheiten enthalten.
Ganz besonders bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.
Insbesondere bevorzugt enthalten die erfindungsgemäßen Reinigungsmittel mindestens ein anionisches Tensid mit der INCI-Bezeichnung Sodium Laureth Sulfate, da diese Tenside eine besonders ausgewogene Balance zwischen Schaumstärke und Milde aufweisen.

Der Gewichtsanteil des oder der anionischen Tensids(e) am Gesamtgewicht der Zusammensetzungen beträgt bevorzugt 0,5 bis 20 Gew.-%, mehr bevorzugt 1 bis 15 Gew.-%, besonders bevorzugt 2 bis 12,5 Gew.-% und insbesondere 3 bis 10 Gew.-%.

Zu den geeigneten amphoteren und/oder zwitterionischen Tensiden die in den erfindungsgemäßen Reinigungsmitteln eingesetzt werden können, zählen beispielsweise eine oder mehrere Verbindungen der nachfolgenden Formeln (I) bis (VII), in denen der Rest R für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht:

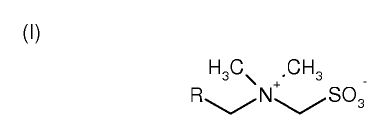

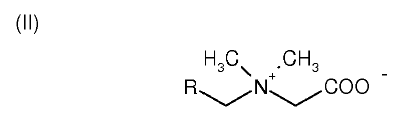

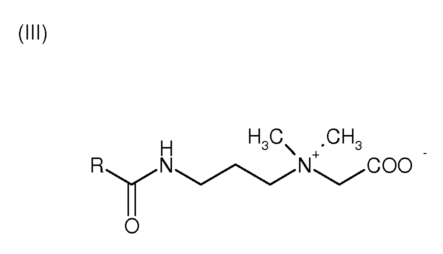

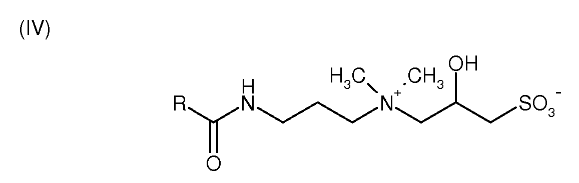

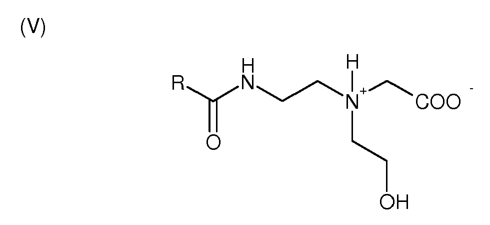

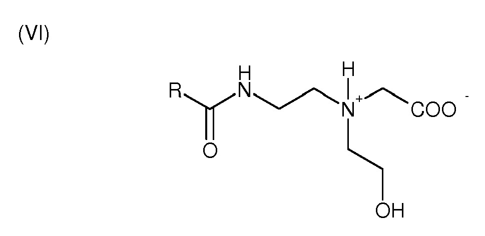

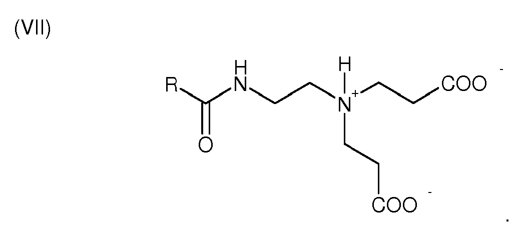

Bevorzugte amphotere und/oder zwitterionische Tenside einer der zuvor genannten Formeln (I) bis (VII) enthalten als Rest R überwiegend einen geradkettigen oder verzweigten, gesättigten, ein- oder mehrfach ungesättigten Alkylrest mit 8 bis 20, mehr bevorzugt von 8 bis 18 und insbesondere mit 8 bis 16 C-Atomen. Besonders bevorzugt sind amphotere und/oder zwitterionische Tenside, bei denen sich der Rest R von Kokosfett ableitet.

Ganz besonders bevorzugt sind die unter den INCI-Bezeichnungen Sodium Cocoamphoacetate, Disodium Cocoamphodiacetate, Sodium Lauroamphoacetate, Sodium Lauroamphodiacetate, Sodium Cocoamphopropionate, Disodium Cocoamphodipropionate, Coco Betaine, Lauryl Betaine Cocamidopropylbetain und/oder Lauramidopropylbetain bekannten und im Handel von mehreren Anbietern erhältlichen amphoteren/zwitterionischen Tenside. Insbesondere bevorzugt sind Tenside mit den INCI-Bezeichnungen Cocoampho(di)acetate und/oder Cocamidopropylbetain.

Der Gewichtsanteil des oder der amphoteren und/oder zwitterionischen Tensids(e) am Gesamtgewicht der Zusammensetzungen beträgt bevorzugt 0,5 bis 20 Gew.-%, mehr bevorzugt 1 bis 15 Gew.-%, besonders bevorzugt 1,25 bis 10 Gew.-% und insbesondere 1,5 bis 7,5 Gew.-%.

Im Rahmen der Erfindung war es erstrebenswert, eine besonders milde und die Kopfhaut schonende Tensidbasis für die Reinigungsmittel zu finden, um die Haare nicht zu belasten. Eine spezielle Mischung aus anionischen und amphoteren Tensiden hat sich für dieses Zweck als besonders geeignet erwiesen.

In einer ersten bevorzugten Ausführungsform enthalten erfindungsgemäße Reinigungsmittel daher bevorzugt
ai) mindestens ein anionisches Tensid aus der Gruppe der Alkylsulfate und/oder Alkylpolyglykolethersulfate der Formel R-O-(CH₂-CH₂O)ₙ-CH₂-CH₂-O-SO₃X, in der R bevorzugt für einen geradkettigen oder verzweigten, gesättigten oder ein- bzw. mehrfach ungesättigten Alkyl- oder Alkenlyrest mit 8 bis 24 Kohlenstoffatomen, n für 0 oder 1 bis 12 und X für ein Alkali- oder Erdalkalimetall oder für Triethanolamin steht, wobei anionische Tenside mit der INCI-Bezeichnung Sodium Laureth Sulfate besonders bevorzugt sind, und
aii) mindestens ein amphoteres (zwitterionisches) Tensid aus der Gruppe der (C₈-C₂₄)-Alkylampho(di)acetate - und/oder -propionate und/oder aus der Gruppe der (C₈-C₂₄)-Alkylamido(C₁-C₄)-alkylbetainen enthält, wobei amphotere (zwitterionische) Tenside mit den INCI-Bezeichnungen Cocoampho(di)acetate und/oder Cocamidopropylbetain besonders bevorzugt sind.

Innerhalb dieser Ausführungsform ist es besonders bevorzugt, wenn die erfindungsgemäßen kosmetischen Reinigungsmittel
ai) mindestens ein anionisches Tensid mit der INCI-Bezeichnung Sodium Laureth Sulfate,
aii) mindestens ein amphoteres (zwitterionisches) Tensid mit der INCI-Bezeichnung Cocoampho(di)acetate und
aiii) mindestens ein amphoteres (zwitterionisches) Tensid mit der INCI-Bezeichnung Cocamidopropylbetain enthalten.

Insbesondere bevorzugte Reinigungsmittel innerhalb dieser Ausführungsform enthalten die Tenside ai), aii) und aiii) in einem Gewichtsverhältnis (ai) : (aii) + aiii))) im Bereich von 5 : 1 bis 1 : 2, bevorzugt von 4,5 : 1 bis 1 : 1, besonders bevorzugt von 4,25 : 1 bis 1,5 : 1 und insbesondere von 4 : 1 bis 2 : 1.

Ein zweiter wesentlicher Bestandteil der erfindungsgemäßen kosmetischen Reinigungsmittel ist ein kationisches Polysaccharid, dessen Gewichtsanteil am Gesamtgewicht der Zusammensetzung vorzugsweise 0,01 bis 5 Gew.-%, mehr bevorzugt 0,05 bis 3 Gew.-%, besonders bevorzugt 0,1 bis 2 Gew.-% und insbesondere 0,2 bis 1 Gew.-% beträgt.

Erfindungsgemäß bevorzugte kationische Polysaccharide sind
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- hydrophob modifizierte Cellulosederivate, beispielsweise die unter dem Handelsnamen SoftCat^{®} vertriebenen kationischen Polymere,
- kationische Alkylpolyglycoside,
- kationisierter Honig, beispielsweise das Handelsprodukt Honeyquat^{®} 50,
- kationische Guar-Derivate, wie insbesondere die unter den Handelsnamen Cosmedia^{®}Guar, N-Hance^{®} und Jaguar^{®} vertriebenen Produkte,

Besonders bevorzugte kationische Polysaccharide sind die kationischen Hydroxyalkyl-Guar-Derivate, vorzugsweise kationisches Hydroxyethyltrimethylammonium-Guar und/oder kationisches Hydroxypropyltrimethylammonium-Guar mit mittleren Molekulargewichten zwischen 100.000 und 2.000.000 Dalton. Insbesondere bevorzugt sind die unter der INCI-Bezeichnung Guar Hydroxypropyltrimonium Chlorid bekannten kationischen Guar-Polymere mit einem Molekulargewicht (Gewichtsmittel) zwischen 200.000 und 1.600.000 Dalton. Die kationische Ladungsdichte dieser Guar-Polymere beträgt vorzugsweise mindestens 0,4 meq/g, bevorzugt mindestens 0,5 meq/g und insbesondere mindestens 0,6 meq/g. Ihr Stickstoffgehalt liegt vorzugsweise im Bereich von 1,1 bis 1,8 Gew.-% (bezogen auf ihr Gesamtgewicht). Zusammenfassend werden erfindungsgemäße kosmetische Zusammensetzungen bevorzugt, die als kationisches Polysaccharid ein kationisches Guar-Derivat enthalten.

Erfindungsgemäß bevorzugte Reinigungsmittel enthalten demnach mindestens ein kationisches Polysaccharid, das ausgewählt ist aus quaternisierten Cellulose-Derivaten, die hydrophob modifiziert sein können, kationischen Alkylpolyglycosiden, kationisiertem Honig und/oder kationischen Guar-Derivaten, wobei kationische Guar-Derivate, insbesondere ein unter der INCI-Bezeichnung Guar Hydroxypropyltrimonium Chloride bekanntes Guar-Derivat, besonders bevorzugt sind.

Ein dritter wesentlicher Bestandteil der erfindungsgemäßen kosmetischen Reinigungsmittel ist ein Silikon, das ausgewählt ist aus wasserunlöslichen, nicht-flüchtigen Polydimethylsiloxanen, die bei 25°C eine Viskosität im Bereich von 30.000 bis 100.000 cSt aufweisen. Der Gewichtsanteil am Gesamtgewicht der Zusammensetzung vorzugsweise 0,01 bis 5 Gew.-%, mehr bevorzugt 0,05 bis 3 Gew.-%, besonders bevorzugt 0,1 bis 2 Gew.-% und insbesondere 0,2 bis 1 Gew.-% beträgt.

Silikone, die in den Reinigungsmitteln eingesetzt werden können, sind
(i) Polyalkylsiloxane, Polyarylsiloxane, Polyalkylarylsiloxane, die flüchtig oder nicht flüchtig, geradkettig, verzweigt oder cyclisch, vernetzt oder nicht vernetzt sein können;
(ii) Polysiloxane, die in ihrer allgemeinen Struktur eine oder mehrere organofunktionelle Gruppen enthalten, die ausgewählt sein können aus:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) lineare Polysiloxan(A)- Polyoxyalkylen(B)- Blockcopoylmere vom Typ (A-B)ₙ mit n > 3;
(iv) gepfropfte Siliconpolymere mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropfte Siliconpolymere mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemische.

Erfindungsgemäß eingesetzte Silikone sind wasserunlösliche, nicht-flüchtige Polydimethylsiloxane, die bei einer Temperatur von 25°C bevorzugt eine Viskosität im Bereich von 30.000 bis 100.000 cSt aufweisen.
Die Viskosität der Polysiloxane kann beispielsweise bei 25°C mit Hilfe eines Glaskapillarviskosimeters nach der Dow Corning Corporate Testmethode CTM0004 (20.07.1970) gemessen werden.

Ein vierter wesentlicher Bestandteil der erfindungsgemäßen kosmetischen Reinigungsmittel ist ein Wachs, dessen Gewichtsanteil am Gesamtgewicht der Zusammensetzung vorzugsweise 0,01 bis 2 Gew.-%, mehr bevorzugt 0,05 bis 1 Gew.-%, besonders bevorzugt 0,075 bis 0,75 Gew.-% und insbesondere 0,1 bis 0,5 Gew.-% beträgt.

Unter geeigneten "Wachsen" sind im Rahmen der Erfindung bevorzugt natürliche und synthetische Substanzen zu verstehen, die üblicherweise die folgenden Eigenschaften aufweisen: bei 20°C knetbar, fest bis brüchig, grob bis feinkristallin, durchscheinend bis opak, jedoch nicht glasartig, über 40°C ohne Zersetzung schmelzend, schon wenig oberhalb des Schmelzpunktes verhältnismäßig niedrigviskos und nicht fadenziehend, stark temperaturabhängige Konsistenz und Löslichkeit, unter leichtem Druck polierbar.

Unter natürlichen Wachsen sind vorzugsweise pflanzliche Wachse wie Carnaubawachs, Candelillawachs und/oder Jojobaöl sowie tierische Wachse wie Bienenwachs, Wollwachs, Walrat und/oder Bürzeldrüsenfett zu verstehen.

Unter synthetischen Wachsen sind vorzugsweise Mineralwachse wie Hartparaffin, Ceresin, Ozokerit, Esterwachse wie Polyethylenglycol- oder Polyethylenglycolesterwachse und/oder gehärtete Pflanzenöle zu verstehen.

Besonders bevorzugt sind chemisch modifizierte (insbesondere gehärtete) oder nicht modifizierte Wachse pflanzlichen Ursprungs, welche bevorzugt Schmelzpunkte im Bereich von 80 bis 90°C, mehr bevorzugt von 82 bis 90°C und insbesondere von 85 bis 88°C aufweisen.

Insbesondere bevorzugt sind gehärtete Pflanzenöle, insbesondere bevorzugt gehärtetes Rizinusöl, das unter der INCI-Bezeichnung Hydrogenated Castor Oil von verschiedenen Anbietern im Handel erhältlich ist.

In einer weiteren bevorzugten Ausführungsform sind erfindungsgemäße kosmetische Reinigungsmittel demnach dadurch gekennzeichnet, dass sie mindestens ein Wachs enthalten, welches einen Schmelzpunkt im Bereich von 80 bis 90°C aufweist, wobei Wachse pflanzlichen Ursprungs bevorzugt sind und gehärtetes Rizinusöl besonders bevorzugt ist.

Ein vierter wesentlicher Bestandteil der erfindungsgemäßen kosmetischen Reinigungsmittel ist mindestens ein natives Öl und mindestens ein kationisches Proteinhydrolysat. Der Gewichtsanteil des nativen Öls am Gesamtgewicht der Zusammensetzung beträgt 0,01 bis 3 Gew.-%, bevorzugt 0,05 bis 2 Gew.-%.

Der Gewichtsanteil des kationischen Proteinhydrolysats am Gesamtgewicht der Zusammensetzung beträgt 0,01 bis 3 Gew.-%, bevorzugt 0,05 bis 2 Gew.-%.

Als native bzw. natürliche (pflanzliche) Öle werden vorzugsweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Marulaöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Besonders bevorzugt sind erfindungsgemäße Reinigungsmittel, die als natives Öl Aprikosenkernöl, Arganöl, Marulaöl, Jojobaöl und/oder Mandelöl enthalten, insbesondere bevorzugt sind Aprikosenkernöl, Marulaöl und Jojobaöl.

Das native Öl liegt in den kosmetischen Reinigungsmitteln vorzugsweise in dispergierter Form vor, wobei die Tröpfchengröße des dispergierten Öls bevorzugt 0,1 bis 5 µm, besonders bevorzugt 0,5 bis 3 µm beträgt.

Unter geeigneten kationischen Proteinhydrolysaten werden vorzugsweise Verbindungen der nachfolgenden Formel (I) verstanden,

R'-X-R" (I),

in der
- R' für einen geradkettigen oder verzweigten, gesättigten oder ungesätttigten Kohlenwasserstoffrest mit 11 bis 24 Kohlenstoffatomen steht,
- R" ein Protein, ein Peptid oder ein Hydrolysat aus Keratin bedeutet,
- X für -C(0)0- oder -N+(R^{III}₂)R^{IV}- oder -N(R^{III})R^{IV}- oder -C(O)-N(R^{V})R^{VI}- steht,
- R^{III} -(CH₂)ₓ-CH₃ mit x = 0 - 22 bedeutet und
- R^{IV}-CH₂-CH(OH)-CH₂- oder -(CH₂)ₓ- mit x = 0 - 22 bedeutet;
- R^{V} und R^{VI} unabhängig voneinander für -H oder -(CH₂)ₓ-CH₃ mit x = 0 - 22 stehen.

Besonders bevorzugt sind Verbindungen der zuvor genannten Formel (I), in denen
- X für -N+(CH₃)₂-CH₂-CH(OH)-CH₂- oder für -C(0)-0-,
- R" für ein Keratinhydrolysat und
- R' für -(CH₂)₁₇-CH₃ steht.

Insbesondere bevorzugt sind kationische Keratinhydrolysate der zuvor genannten Formel (I), die ein mittleres Molgewicht (Gewichtsmittel) im Bereich von 500 bis 10000 Da, vorzugsweise von 750 bis 5000 Da, besonders bevorzugt von 750 bis 2000 Da und insbesondere von 800 bis 1500 Da aufweisen.
Es ist weiterhin bevorzugt, wenn der Rest R" für kationische Keratinhydrolysate steht, die kein Methionin enthalten, und besonders bevorzugt, wenn der R" für kationische Keratinhydrolysate steht, die große Mengen an Cystein und/oder Cystin enthalten.
Beispiele für besonders bevorzugte Handelsprodukte sind beispielsweise die Produkte Croquat^{®} WKP oder Promois^{®} WK-HCAQ, die unter der INCI-Bezeichnung Cocodimonium Hydroxypropyl Hydrolyzed Keratin erhältlich sind.

Insbesondere bevorzugte kationische Proteinhydrolysate gemäß der zuvor genannten Formel sind die unter der INCI-Bezeichnung Cocodimonium Hydroxypropyl Hydrolyzed Keratin bekannten Verbindungen.

In einer weiteren bevorzugten Ausführungsform sind erfindungsgemäße kosmetische Reinigungsmittel demnach dadurch gekennzeichnet, dass sie mindestens ein kationisches Proteinhydrolysat enthalten, das ausgewählt ist aus Verbindungen der allgemeinen Formel (I),

R'-X-R" (I),

in der
- R' für einen geradkettigen oder verzweigten, gesättigten oder ungesättigten Kohlenwasserstoffrest mit 8 bis 24 Kohlenstoffatomen steht,
- R" ein Protein oder ein Proteinhydrolysat bedeutet,
- X für -C(0)0- oder -N⁺(R^{III} ₂)R^{IV}- oder -N(R^{III})R^{IV}- oder-C(O)-N(R^{V})R^{VI}- steht,
- R^{III} -(CH₂)ₓ-CH₃ mit x = 0 - 22 bedeutet,
- R^{IV} -CH₂-CH(OH)-CH₂- oder -(CH₂)ₓ- mit x = 0 - 22 bedeutet, und
- R^{V} und R^{V} unabhängig voneinander für -H oder -(CH₂)ₓ-CH₃ mit x = 0 - 22 stehen,
wobei kationische Proteinhydrolysate nach Formel (I) besonders bevorzugt sind, in denen
- X für -N+(CH₃)₂-CH₂-CH(OH)-CH₂- oder für -C(0)-0-,
- R" für ein Keratinhydrolysat und
- R' für -(CH₂)₁₇-CH₃ steht, und
wobei ein unter der INCI-Bezeichnung Cocodimonium Hydroxypropyl Hydrolyzed Keratin bekanntes kationisches Proteinhydrolysat insbesondere bevorzugt ist.

Es wurde gefunden, dass die Haarstruktur in besonderem Maße gestärkt werden kann, und dass Spliss und Haarbruch signifikant reduziert werden können, wenn die erfindungsgemäßen Reinigungsmittel neben den speziellen Tensiden, dem kationischen Polysaccharid, dem Silikon und dem Wachs eine Kombination aus nativem Öl und einem kationischen Proteinhydrolysat enthalten.

In einer zweiten besonders bevorzugten Ausführungsform enthalten erfindungsgemäße kosmetische Reinigungsmittel demnach bevorzugt mindestens ein natives Öl und mindestens ein kationisches Proteinhydrolysat gemäß der zuvor genannten Formel (I).

Der Pflegeeffekt der erfindungsgemäßen Reinigungsmittel ist besonders hoch und insbesondere der Haarbruch konnte deutlich vermindert und/oder beseitigt werden, wenn das native Öl und das kationische Proteinhydrolysat in einem bestimmten Gewichtsverhältnis eingesetzt werden.

Innerhalb dieser zweiten Ausführungsform sind daher solche erfindungsgemäßen Reinigungsmittel besonders bevorzugt, die mindestens ein natives Öl (O)und mindestens ein kationisches Proteinhydrolysat (P) in einem Gewichtsverhältnis O : P von 5 : 1 bis 1 : 5, mehr bevorzugt von 3 : 1 bis 1 : 3 und insbesondere von 2 : 1 bis 1 : 2 enthalten.

Insbesondere bevorzugte erfindungsgemäße Reinigungsmittel innerhalb dieser Ausführungsform enthalten
- kationische Proteinhydrolysate nach Formel (I) (P), in denen
   - X für -N+(CH₃)₂-CH₂-CH(OH)-CH₂- oder für -C(O)-O-,
   - R" für ein Keratinhydrolysat und
   - R' für -(CH₂)₁₇-CH₃ steht, besonders bevorzugt ein unter der INCI-Bezeichnung Cocodimonium Hydroxypropyl Hydrolyzed Keratin bekanntes kationisches Proteinhydrolysat nach Formel (I), und
- mindestens ein natives Öl (O), ausgewählt aus Aprikosenkernöl, Arganöl, Marulaöl, Jojobaöl und/oder Mandelöl, besonders bevorzugt Aprikosenkernöl, Marulaöl und/oder Jojobaöl und insbesondere Marulaöl,
in einem Gewichtsverhältnis O : P von 3 : 1 bis 1 : 3, bevorzugt 2 : 1 bis 1 : 2 und insbesondere 1,5 : 1 bis 1 : 1.

Ein Ziel der vorliegenden Erfindung war es kosmetische Reinigungsmittel bereitzustellen, die eine optimale Balance zwischen gründlicher aber die Haare und die Kopfhaut schonender Reinigung und hervorragender Pflege aufweisen. Unter "Pflege" wird insbesondere die Kräftigung der Haarstruktur sowie die Beseitigung, Verminderung oder Vorbeugung von Spliss oder Haarbruch, insbesondere von Haarbruch verstanden. Es wurde festgestellt, dass eine optimale Balance zwischen Reinigung und Pflege erreicht werden kann, wenn die erfindungsgemäßen Reinigungsmittel die wesentlichen Inhaltsstoffe im Wesentlichen in üblichen Mengen enthalten. Besonders gute Ergebnisse konnten jedoch erzielt werden, wenn die erfindungsgemäßen Reinigungsmittel die wesentlichen Inhaltsstoffe a) bis e) in eingeengten Mengenbereichen enthalten.

In einer dritten bevorzugten Ausführungsform sind erfindungsgemäß bevorzugte kosmetische Reinigungsmittel demnach dadurch gekennzeichnet, dass
a) der Gewichtsanteil des anionischen und/oder amphoteren (zwitterionischen) Tensids am Gesamtgewicht der Zusammensetzung jeweils 0,5 bis 20 Gew.-%, vorzugsweise von 1 bis 15 Gew.-%,
b) der Gewichtsanteil des kationischen Polysaccharids am Gesamtgewicht der Zusammensetzung 0,01 bis 5 Gew.-%, vorzugsweise 0,05 bis 3 Gew.-%,
c) der Gewichtsanteil des Silikons am Gesamtgewicht der Zusammensetzung 0,01 bis 5 Gew.-%, vorzugsweise 0,05 bis 3 Gew.-%,
d) der Gewichtsanteil des Waches am Gesamtgewicht der Zusammensetzung 0,01 bis 2 Gew.-%, vorzugsweise 0,05 bis 1 Gew.-%,
e) der Gewichtsanteil des nativen Öls am Gesamtgewicht der Zusammensetzung 0,01 bis 3 Gew.-%, vorzugsweise 0,05 bis 2 Gew.-%, und
f) der Gewichtsanteil des kationisches Proteinhydrolysats am Gesamtgewicht der Zusammensetzung 0,01 bis 3 Gew.-%, vorzugsweise 0,05 bis 2 Gew.-%, beträgt.

Innerhalb dieser Ausführungsform sind solche erfindungsgemäßen Reinigungsmittel besonders bevorzugt, die
a) 0,5 bis 20 Gew.-%, mehr bevorzugt 1 bis 15 Gew.-% Alkylsulfate und/oder Alkylpolyglykolethersulfatsalze der Formel R-O-(CH₂-CH₂O)ₙ-CH₂-CH₂-O-SO₃X, in der R bevorzugt für einen geradkettigen oder verzweigten, gesättigten oder ein- bzw. mehrfach ungesättigten Alkyl- oder Alkenlyrest mit 8 bis 24 Kohlenstoffatomen, n für 0 oder 1 bis 12 und X für ein Alkali- oder Erdalkalimetall oder für Triethanolamin steht,
b) 0,5 bis 20 Gew.-%, mehr bevorzugt 1 bis 15 Gew.-% mindestens eines der unter den INCI-Bezeichnungen Sodium Cocoamphoacetate, Disodium Cocoamphodiacetate, Sodium Lauroamphoacetate, Sodium Lauroamphodiacetate, Sodium Cocoamphopropionate, Disodium Cocoamphodipropionate, Coco Betaine, Lauryl Betaine Cocamidopropylbetain und/oder Lauramidopropylbetain bekannten amphoteren/zwitterionischen Tenside,
c) 0,01 bis 5 Gew.-%, mehr bevorzugt 0,05 bis 3 Gew.-% mindestens eines kationischen Hydroxyalkyl-Guar-Derivats,
d) 0,01 bis 5 Gew.-%, mehr bevorzugt 0,05 bis 3 Gew.-% mindestens eines Polydialkylsiloxans, besonders bevorzugt eines Polydimethylsiloxans,
e) 0,01 bis 2 Gew.-%, mehr bevorzugt 0,05 bis 1 Gew.-% mindestens ein Waches pflanzlichen Ursprungs, welches einen Schmelzpunkt im Bereich von 80 bis 90°C aufweist,
f) 0,01 bis 3 Gew.-%, mehr bevorzugt 0,05 bis 2 Gew.-% mindestens eines nativen Öls und
g) 0,01 bis 3 Gew.-%, mehr bevorzugt 0,05 bis 2 Gew.-% mindestens eines kationischen Proteinhydrolysats nach Formel (I) enthalten,
wobei sich die Mengenangaben auf den Gewichtsanteil der jeweiligen Komponente(n) am Gesamtgewicht der Zusammensetzungen beziehen.

Innerhalb dieser Ausführungsform sind solche erfindungsgemäßen Reinigungsmittel insbesondere bevorzugt, die
a) 0,5 bis 20 Gew.-%, mehr bevorzugt 1 bis 15 Gew.-% mindestens eines anionischen Tensids mit der INCI-Bezeichnung Sodium Laureth Sulfate,
b) 0,5 bis 20 Gew.-%, mehr bevorzugt 1 bis 15 Gew.-% mindestens eines der unter den INCI-Bezeichnungen Sodium Cocoamphoacetate, Disodium Cocoamphodiacetate, und/oder Cocamidopropylbetain bekannten amphoteren/zwitterionischen Tenside,
c) 0,01 bis 5 Gew.-%, mehr bevorzugt 0,05 bis 3 Gew.-% mindestens eines unter der INCI-Bezeichnung Guar Hydroxypropyltrimonium Chloride bekannten kationischen Polysaccharids,
d) 0,01 bis 5 Gew.-%, mehr bevorzugt 0,05 bis 3 Gew.-% mindestens eines Polydimethylsiloxans, das bei 25°C eine Viskosität im Bereich von 1.000 bis 1.000.000 cSt, bevorzugt von 5.000 bis 500.000 cSt, besonders bevorzugt von 10.000 bis 200.000 cSt und insbesondere von 30.000 bis 100.000 cSt aufweist,
e) 0,01 bis 2 Gew.-%, mehr bevorzugt 0,05 bis 1 Gew.-% gehärtetes Rizinusöl (INCI-Bezeichnung: Hydrogenated Castor Oil),
f) 0,01 bis 3 Gew.-%, mehr bevorzugt 0,05 bis 2 Gew.-% Aprikosenkernöl, Arganöl, Marulaöl, Jojobaöl und/oder Mandelöl, und
g) 0,01 bis 3 Gew.-%, mehr bevorzugt 0,05 bis 2 Gew.-% mindestens eines unter der INCI-Bezeichnung Cocodimonium Hydroxypropyl Hydrolyzed Keratin bekannten kationischen Proteinhydrolysats nach Formel (I) enthalten,
wobei sich die Mengenangaben auf den Gewichtsanteil der jeweiligen Komponente(n) am Gesamtgewicht der Zusammensetzungen beziehen.

Neben den zuvor genannten Wirk- und Trägerstoffen können die erfindungsgemäßen kosmetischen Reinigungsmittel noch eine Reihe weiterer Inhaltsstoffe enthalten, die ihnen vorteilhafte Eigenschaften verleihen. Zu den bevorzugten fakultativen Wirkstoffen, die in den erfindungsgemäßen Zusammensetzungen eingesetzt werden können, zählen beispielsweise:
- nichtionische Tenside und/oder nichtionische Emulgatoren, die in den kosmetischen Reinigungsmitteln (bezogen auf das Gesamtgewicht der Reinigungsmittel) bevorzugt in einer Menge von 0,1 bis 10 Gew.-%, mehr bevorzugt 0,25 bis 7,5 Gew.-% und insbesondere 0,5 bis 5 Gew.-% eingesetzt werden können,
- weitere Öl-, Wachs- und/oder Fettkomponenten, die in den kosmetischen Reinigungsmitteln (bezogen auf das Gesamtgewicht der Reinigungsmittel) bevorzugt in einer Menge von 0,01 - 10 Gew.-%, besonders bevorzugt von 0,05 - 7,5 Gew.% und insbesondere von 0,1 - 5 Gew.% eingesetzt werden können,
- Antischuppenwirkstoffe, die in den in den kosmetischen Reinigungsmitteln (bezogen auf das Gesamtgewicht der Reinigungsmittel) bevorzugt in einer Menge von 0,01 bis 7,5 Gew.-%, besonders bevorzugt von 0,05 bis 5 Gew.-% und insbesondere von 0,1 bis 3 Gew.-% eingesetzt werden können.

Zu den geeigneten nichtionischen Tensiden/Emulgatoren zählen beispielsweise
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Fettsäurealkanolamide, insbesondere C₈-C₂₄-Fettsäuremono-(C₂-C₄)-alkanolamide und C₈-C₂₄-Fettsäuredi-(C₂-C₄)-alkanolamide
- Anlagerungsprodukte von Ethylenoxid an Fettamine und/oder
- Alkylpolyglucoside.

Besonders geeignete nichtionische Tenside sind Alkyloligoglucoside, insbesondere Alkyloligoglucoside auf der Basis von gehärtetem C_{12/14}-Kokosalkohol mit einem DP von 1-3, wie sie beispielsweise unter der INCI-Bezeichnung "Coco-Glucoside", "Decyl Glucoside" und/oder "Lauryl Glucoside" im Handel erhältlich sind, sowie Fettsäurealkanolamide, bevorzugt C₈-C₂₄-Fettsäuremono-(C₂-C₄)-alkanolamide und insbesondere die unter den INCI-Bezeichnungen Cocamide MEA, Lauramid MEA, Palmitoylamid MEA, Cocamide MIPA und Lauramid MIPA bekannten Verbindungen. Weiterhin besonders bevorzugte nichtionische Tenside sind die C₈-C₃₀-Fettsäuremono- und -diester von Anlagerungsprodukten von 1 bis 30 Mol Ethylenoxid an Glycerin. Besonders bevorzugt sind die C₁₀-C₁₆-Fettsäuremono- und -diester von Anlagerungsprodukten von 1 bis 10 Mol Ethylenoxid an Glycerin. Insbesondere bevorzugt ist die unter der INCI-Bezeichnung PEG-7 Glyceryl Cocoate bekannte Verbindung.

In einer weiteren besonders bevorzugten Ausführungsform enthalten die erfindungsgemäßen Reinigungsmittel zusätzlich zu den Wirkstoffen a) bis e) mindestens ein nichtionisches Tensid, ausgewählt aus Alkylpolyglycosiden, C₈-C₂₄-Fettsäuremono-(C₂-C₄)-alkanolamiden und/oder C₁₀-C₁₆-Fettsäuremono- und -diestern von Anlagerungsprodukten von 1 bis 10 Mol Ethylenoxid an Glycerin.

Geeignete weitere Öl- und/oder Fettkomponenten können bevorzugt ausgewählt sein aus mineralischen Ölkomponenten und/oder Fettstoffen.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S).
Als Ölkomponente kann weiterhin ein Dialkylether dienen. Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether. Besonders bevorzugt ist der Di-n-octylether, der im Handel unter der Bezeichnung Cetiol^{®} OE erhältlich ist.

Unter Fettstoffen sind zu verstehen Fettsäuren, Fettalkohole sowie natürliche und synthetische Wachse, welche sowohl in fester Form als auch flüssig in wässriger Dispersion vorliegen können.
Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
Besonders bevorzugt sind üblicherweise die Fettsäureschnitte, welche aus Cocosöl oder Palmöl erhältlich sind; insbesondere bevorzugt ist in der Regel der Einsatz von Stearinsäure.
Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.
Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol- caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD.

Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten. Besonders bevorzugt sind Climbazol, Zink Pyrithion und Piroctone Olamine, insbesondere bevorzugt ist Zink Pyrithion.

Zu den weiteren fakultativen Komponenten, die in den erfindungsgemäßen Reinigungsmitteln eingesetzt werden können, zählen beispielsweise
- Vitamine, Vitaminderivate und/oder Vitaminvorstufen,
- Pflanzenextrakte und/oder
- Feuchthaltemittel.

Unter geeigneten Vitaminen sind bevorzugt die folgenden Vitamine, Provitamine und Vitaminvorstufen sowie deren Derivate zu verstehen:
Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
Vitamin B: zur Vitamin B-Gruppe oder zu dem Vitamin B-Komplex gehören u. a.
   - Vitamin B₄ (Thiamin)
   - Vitamin B₂ (Riboflavin)
   - Vitamin B₃. Unter dieser Bezeichnung werden häufig die Verbindungen Nicotinsäure und Nicotinsäureamid (Niacinamid) geführt.
   - Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate.
   - Vitamin B₆ (Pyridoxin sowie Pyridoxamin und Pyridoxal).
Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
Vitamin E (Tocopherole, insbesondere α-Tocopherol).
Vitamin F: unter dem Begriff "Vitamin F" werden üblicherweise essentielle Fettsäuren, insbesondere Linolsäure, Linolensäure und Arachidonsäure, verstanden.
Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.
Die erfindungsgemäßen kosmetischen Reinigungsmittel können bevorzugt Vitamine, Provitamine und Vitaminvorstufen aus den Gruppen A, B, E und H enthalten.
Insbesondere bevorzugt sind Nicotinsäureamid, Biotin, Pantolacton und/oder Panthenol.
Vitamine, Vitaminderivate und/oder Vitaminvorstufen können in den erfindungsgemäßen (bezogen auf das Gesamtgewicht der Reinigungsmittel) jeweils bevorzugt in einer Menge von 0,001 bis 5 Gew.-%, bevorzugt 0,005 bis 3 Gew.-% und insbesondere 0,01 bis 2 Gew.-% eingesetzt werden.

Unter geeigneten Pflanzenextrakten sind Extrakte zu verstehen, die aus allen Teilen einer Pflanze hergestellt werden können. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Geeignet sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Boerhavia Diffusa-Wurzeln, Foeniculum vulgaris und Apim graveolens.
Besonders bevorzugt für die Verwendung in den erfindungsgemäßen Zusammensetzungen sind die Extrakte aus Grünem Tee, Brennessel, Hamamelis, Kamille, Aloe Vera, Ginseng, Echinacea purpurea, Olea europea und/oder Boerhavia Diffusa-Wurzeln.
Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.
Die Pflanzenextrakte können sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.
Die Pflanzenextrakte können in den erfindungsgemäßen Reinigungsmitteln (bezogen auf das Gesamtgewicht der Reinigungsmittel) bevorzugt in einer Menge von 0,01 bis 10 Gew.-%, mehr bevorzugt von 0,05 bis 7,5 Gew.-% und insbesondere von 0,1 bis 5 Gew.-% eingesetzt werden.

Geeignete Feuchthaltemittel bzw. Penetrationshilfsstoffe und/ oder Quellmittel, die den erfindungsgemäßen Reinigungsmitteln zugesetzt werden können, sind beispielsweise Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.
Die Feuchthaltemittel können in den erfindungsgemäßen Reinigungsmitteln - bezogen auf deren Gesamtgewicht - bevorzugt in Mengen von 0,01 bis 10 Gew.-%, mehr bevorzugt in Mengen von 0,05 bis 5 Gew.-% und insbesondere in Mengen von 0,1 bis 3 Gew.-% eingesetzt werden.

Weitere Wirk-, Hilfs- und Zusatzstoffe, die in den erfindungsgemäßen Reinigungsmitteln eingesetzt werden können, sind beispielsweise:
- UV-Filter,
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure,
- Wirkstoffe wie Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Perlglanzmittel wie EGDS oder PEG-3 Distearate,
- Konservierungsmittel, wie beispielsweise Natriumbenzoat oder Salicylsäure,
- Viskositätsregler wie Salze (NaCl).

Die erfindungsgemäßen kosmetischen Zusammensetzungen weisen bevorzugt pH-Werte im Bereich von 2,5 bis 6,5, bevorzugt von 3,5 bis 6 und insbesondere von 4 bis 5,5 auf.

Wie eingangs ausgeführt, weisen die erfindungsgemäßen Reinigungsmittel hervorragende Eigenschaften in der Anwendung auf den Haaren auf. Neben der schonenden und gründlichen Reinigung verleihen sie den damit behandelten Haaren verbesserte Eigenschaften. Behandelte trockene Haare (insbesondere vorher in ihrer Struktur geschädigte Haare und brüchige Haare) weisen neben signifikant weniger Haarbruch einen verbesserten Haargriff sowie mehr Glätte, Glanz und Weichheit auf. Behandelte nasse Haare (insbesondere vorher in ihrer Struktur geschädigte Haare und brüchige Haare) weisen neben signifikant weniger Haarbruch weniger bzw. keine Verknotungen auf. Ein weiterer Vorteil der erfindungsgemäßen Reinigungsmittel ist, dass sie die Widerstandfähigkeit der Haaroberfläche gegen physikalische und/oder chemische Haarschädigungen unterstützen.

Ein zweiter Gegenstand der vorliegenden Anmeldung ist die Verwendung eines erfindungsgemäßen kosmetischen Reinigungsmittels zur Beseitigung, Verminderung und/oder Vermeidung von Haarbruch.

Ein dritter Gegenstand der vorliegenden Anmeldung ist ein Verfahren zur Reinigung von Haaren und/oder zur Beseitigung, Verminderung und/oder Vermeidung von Haarbruch, bei dem ein kosmetisches Mittel, enthaltend
a) mindestens ein anionisches und/oder amphoteres (zwitterionisches) Tensid,
b) mindestens ein kationisches Polysaccharid,
b) mindestens eine Silikon, das ausgewählt ist aus wasserunlöslichen, nicht-flüchtigen Polydimethylsiloxanen, die bei 25°C eine Viskosität im Bereich von 30.000 bis 100.000 cSt aufweisen,
c) mindestens ein Wachs, und
d) mindestens ein natives Öl und mindestens ein kationisches Proteinhydrolysat, beide in Mengen bezogen auf das Gesamtgewicht der Zusammensetzung von 0,01 bis 3 Gew.-%,
auf die vorzugsweise nassen Haare aufgebracht, und nach einer Einwirkungszeit von 5 Sekunden bis 5 Minuten mit Wasser ausgespült wird.

Bezüglich bevorzugter Ausführungsformen der erfindungsgemäßen Verwendung sowie bezüglich des erfindungsgemäßen Verfahrens gilt mutatis mutandis das zu der erfindungsgemäßen Verwendung Gesagte.

Die nachfolgenden Beispiele sollen den Gegenstand der Erfindung verdeutlichen, ohne ihn darauf zu beschränken.

### Beispiele:

### 1) Herstellung erfindunasaemäßer Shampoos (Mengen in [Gew.-%]):

### (Beispiel 4 nicht erfindungsgemäß)

| | **Bsp. 1** | **Bsp. 2** | **Bsp. 3** | **Bsp. 4** | **Bsp. 5** |
|---|---|---|---|---|---|
| **Sodium Laureth Sulfate (AS)** | 9 | 9 | 8 | 10 | |
| **Disodium Cocoamphodiacetate (AS)** | 0,8 | | 1 | 0,5 | 3 |
| **Cocoamidopropyl Betaine (AS)** | 1,6 | 2,5 | 2 | 1,5 | 4,5 |
| **Cocamide MEA** | 0,45 | 0,5 | | 0,35 | 0,7 |
| **PEG-7 Glyceryl Cocoate** | 0,4 | 0,6 | 0,5 | | 0,7 |
| **PEG-40 Hydrogenated Castor Oil** | 0,3 | 0,3 | 0,3 | 0,4 | 0,4 |
| **Guar Hydroxypropyltrimonium Chloride** | 0,3 | 0,3 | 0,2 | 0,2 | 0,4 |
| **Polydimethylsiloxane 60000 cSt** | 0,4 | 0,4 | 0,3 | 0,3 | 0,5 |
| **Marulaöl** | 0,05 | 0,05 | | | 0,05 |
| **Jojobaöl** | | | 0,1 | | |
| **Aprikosenkernöl** | | | | 0,1 | |
| **Hydrogenated Castor Oil** | 0,1 | 0,15 | 0,2 | 0,1 | 0,1 |
| **Cocodimonium Hydroxypropyl Hydrolyzed Keratin** | 0,05 | 0,05 | 0,05 | | 0,05 |
| **Natriumbenzoat** | 0,5 | 0,5 | 0,5 | 0,5 | 0,5 |
| **Zitronensäure** | 0,1-1,5 | 0,1-1,5 | 0,1-1,5 | 0,1-1,5 | 0,1-1,5 |
| **NaCl** | 0,1-2,5 | 0,1-2,5 | 0,1-2,5 | 0,1-2,5 | 0,1-2,5 |
| **Parfum** | 0,5-1 | 0,5-1 | 0,5-1 | 0,5-1 | 0,5-1 |
| **Wasser** | ad 100 | ad 100 | ad 100 | ad 100 | ad 100 |
| **pH-Wert** | 4,5-5 (±0,2) | 4,5-5 (±0,2) | 4,5-5 (±0,2) | 4,5-5 (±0,2) | 4,5-5 (±0,2) |
| **Viskosität (Brookfield Viskosimeter, 20°C, 20 U/min) [mPas]** | 6000 - 9000 | 6000 - 9000 | 6000 - 9000 | 6000 - 9000 | 6000 - 9000 |

### 2) Beurteilung erfindungsgemäßer Shampoos:

Es wurden zwei Zusammensetzungen hergestellt. Eine erfindungsgemäße Zusammensetzung entsprach Beispiel 1 (A) der zuvor genannten Tabelle und eine zweite, nicht erfindungsgemäße Zusammensetzung entsprach einem handelsüblichen Pflegeshampoo für brüchige und splissanfällige Haare (B), das anstatt des erfindungsgemäßen Pflegekomplexes, bestehend aus Guar Hydroxypropyltrimonium Chloride, Polydimethylsiloxan, Marulaöl, Hydrogenated Castor Oil und Cocodimonium Hydroxypropyl Hydrolyzed Keratin, 0,7 Gew.-% Polymer JR 400 und 0,2 Gew.-% Polydimethylsiloxan enthielt.

Die beiden Zusammensetzungen wurden auf identisch vorbehandelten (gereinigten und anschließend vorgeschädigten) Haarsträhnen der Firma Kerling International (Backnang, Deutschland - European natural hair 7/0; lot #04/2010; N74; Länge: 12 cm; Gewicht: 1 +/- 0,05g) in üblicher Weise angewendet und anschließend die Reduktion des Haarbruchs bestimmt.

Die Ergebnisse können der nachfolgenden Tabelle entnommen werden:

| | **Reduktion des Haarbruchs [%]** |
|---|---|
| **Shampoo gemäß Beispiel 1 (A)** | 94 |
| **Vergleichsshampoo (B)** | 51 |

Die Ergebnisse in der Tabelle zeigen eindeutig, dass die erfindungsgemäßen Reinigungsmittel signifikant besser gegen den Haarbruch bekämpfen als vergleichbare Shampoos aus dem Stand der Technik.

## Patentansprüche

1. Kosmetisches Reinigungsmittel, enthaltend in einem kosmetischen Träger
a) mindestens ein anionisches und/oder amphoteres (zwitterionisches) Tensid,
b) mindestens ein kationisches Polysaccharid,
c) mindestens eine Silikon, das ausgewählt ist aus wasserunlöslichen, nicht-flüchtigen Poliydimethyisiloxanen, die bei 25°C eine Viskosität im Bereich von 30.000 bis 100.000 cSt aufweisen
d) mindestens ein Wachs,
e) mindestens ein natives Öl und
f) mindestens ein kationisches Proteinhydrolysat, wobei der Gewichtsanteil des kationischen Proteinhydrolysats am Gesamtgewicht der Zusammensetzung 0,01 bis 3 Gew.-% beträgt, wobei der Gewichtsanteil des nativen Öls am Gesamtgewicht der Zusammensetzung 0,01 bis 3 Gew.-% beträgt.

2. Kosmetisches Reinigungsmittel nach Anspruch 1, **dadurch gekennzeichnet, dass** es
ai) mindestens ein anionisches Tensid aus der Gruppe der Alkylsulfate und/oder Alkylpolyglykolethersulfate der Formel R-O-(CH₂-CH₂O)ₙ-CH₂-CH₂-O-SO₃X enthält, in der R bevorzugt für einen geradkettigen oder verzweigten, gesättigten oder ein- bzw. mehrfach ungesättigten Alkyl- oder Alkenlyrest mit 8 bis 24 Kohlenstoffatomen, n für 0 oder 1 bis 12 und X für ein Alkali- oder Erdalkalimetall oder für Triethanolamin steht, wobei anionische Tenside mit der INCI-Bezeichnung Sodium Laureth Sulfate besonders bevorzugt sind, und
aii) mindestens ein amphoteres (zwitterionisches) Tensid aus der Gruppe der (C₈-C₂₄)-Alkylampho(di)acetate - und/oder -propionate und/oder aus der Gruppe der (C₈-C₂₄)-Alkylamido(C₁-C₄)-alkylbetainen enthält, wobei amphotere (zwitterionische) Tenside mit den INCI-Bezeichnungen Cocoampho(di)acetate und/oder Cocamidopropylbetain besonders bevorzugt sind.

3. Kosmetisches Reinigungsmittel nach einem der vorherigen Ansprüche, **dadurch gekennzeichnet, dass** das kationische Polysaccharid ausgewählt ist aus quaternisierten Cellulose-Derivaten, die hydrophob modifiziert sein können, kationischen Alkylpolyglycosiden, kationisiertem Honig und/oder kationischen Guar-Derivaten, wobei kationische Guar-Derivate, insbesondere das unter der INCI-Bezeichnung Guar Hydroxypropyltrimonium Chloride bekannte Guar-Derivat, besonders bevorzugt sind.

4. Kosmetisches Reinigungsmittel nach einem der vorherigen Ansprüche, **dadurch gekennzeichnet, dass** es mindestens ein Wachs enthält, welches einen Schmelzpunkt im Bereich von 80 bis 90°C aufweist, wobei Wachse pflanzlichen Ursprungs bevorzugt sind und gehärtetes Rizinusöl besonders bevorzugt ist.

5. Kosmetisches Reinigungsmittel nach einem der vorherigen Ansprüche, **dadurch gekennzeichnet, dass** das kationische Proteinhydrolysat ausgewählt ist aus Verbindungen der allgemeinen Formel (I),
R'-X-R" (I),
in der
- R' für einen geradkettigen oder verzweigten, gesättigten oder ungesättigten Kohlenwasserstoffrest mit 8 bis 24 Kohlenstoffatomen steht,
- R" ein Protein oder ein Proteinhydrolysat bedeutet,
- X für -C(O)O- oder -N⁺(R^{III}₂)R^{IV}- oder -N(R^{III})R^{IV}- oder -C(O)-N(R^{V})R^{VI}- steht,
- R^{III} -(C^{H}₂)ₓ-CH₃ mit x = 0 - 22 bedeutet,
- R^{IV} -CH₂-CH(OH)-CH₂- oder -(CH₂)ₓ- mit x = 0 - 22 bedeutet, und
- R^{V} und R^{V} unabhängig voneinander für -H oder -(C^{H}₂)ₓ-CH₃ mit x = 0 - 22 stehen,
wobei kationische Proteinhydrolysate nach Formel (I) bevorzugt sind, in denen
- X für -N⁺(CH₃)₂-CH₂-CH(OH)-CH₂- oder für -C(O)-O-,
- R" für ein Keratinhydrolysat und
- R' für -(CH₂)₁₇-CH₃ steht, und
wobei ein unter der INCI-Bezeichnung Cocodimonium Hydroxypropyl Hydrolyzed Keratin bekanntes kationisches Proteinhydrolysat besonders bevorzugt ist.

6. Kosmetisches Reinigungsmittel nach einem der vorhergehenden Ansprüche, **dadurch gekennzeichnet, dass**
a) der Gewichtsanteil des anionischen und/oder amphoteren (zwitterionischen) Tensids am Gesamtgewicht der Zusammensetzung jeweils 0,5 bis 20 Gew.-%, vorzugsweise von 1 bis 15 Gew.-%,
b) der Gewichtsanteil des kationischen Polysaccharids am Gesamtgewicht der Zusammensetzung 0,01 bis 5 Gew.-%, vorzugsweise 0,05 bis 3 Gew.-%,
c) der Gewichtsanteil des Silikons am Gesamtgewicht der Zusammensetzung 0,01 bis 5 Gew.-%, vorzugsweise 0,05 bis 3 Gew.-%,
d) der Gewichtsanteil des Waches am Gesamtgewicht der Zusammensetzung 0,01 bis 2 Gew.-%, vorzugsweise 0,05 bis 1 Gew.-%,
e) der Gewichtsanteil des nativen Öls am Gesamtgewicht der Zusammensetzung 0,01 bis 3 Gew.-%, vorzugsweise 0,05 bis 2 Gew.-%, und
f) der Gewichtsanteil des kationischen Proteinhydrolysats am Gesamtgewicht der Zusammensetzung 0,01 bis 3 Gew.-%, vorzugsweise 0,05 bis 2 Gew.-% beträgt.

7. Verwendung eines kosmetischen Reinigungsmittels nach einem der Ansprüche 1 bis 6 zur Beseitigung, Verminderung und/oder Vermeidung von Haarbruch.

8. Verfahren zur Reinigung von Haaren und/oder zur Beseitigung, Verminderung und/oder Vermeidung von Haarbruch, bei dem ein kosmetisches Mittel, enthaltend
a) mindestens ein anionisches und/oder amphoteres (zwitterionisches) Tensid,
b) mindestens ein kationisches Polysaccharid,
c) mindestens eine Silikon, das ausqewählt ist aus wasserunlöslichen, nicht-flüchtigen Polydimethylsiloxanen, die bei 25°C eine Viskosität im Bereich von 30.000 bis 100.000 cSt aufweisen
d) mindestens ein Wachs,
e) mindestens ein natives Öl und
f) mindestens ein kationisches Proteinhydrolysat
auf die vorzugsweise nassen Haare aufgebracht, und nach einer Einwirkungszeit von 5 Sekunden bis 5 Minuten mit Wasser ausgespült wird, wobei der Gewichtsanteil des kationischen Proteinhydrolysats am Gesamtgewicht der Zusammensetzung 0,01 bis 3 Gew.-% beträgt, wobei der Gewichtsanteil des nativen Öls am Gesamtgewicht der Zusammensetzung 0,01 bis 3 Gew.-% beträgt.

## Claims

1. A cosmetic cleaning agent containing, in a cosmetic carrier
a) at least one anionic and/or amphoteric (zwitterionic) surfactant,
b) at least one cationic polysaccharide,
c) at least one silicone that is selected from water-insoluble, non-volatile polydimethyl siloxanes, which have a viscosity at 25 °C in the range of from 30,000 to 100,000 cSt,
d) at least one wax,
e) at least one native oil and
f) at least one cationic protein hydrolyzate, wherein the weight proportion of the cationic protein hydrolyzate in the total weight of the composition is 0.01 to 3 wt%, wherein the weight proportion of the native oil in the total weight of the composition is 0.01 to 3 wt%.

2. The cosmetic cleaning agent according to claim 1, **characterized in that** it contains
ai) at least one at least one anionic surfactant from the group of alkyl sulfates and/or alkyl polyglycol ether sulfates of formula R-O-(CH₂-CH₂0)ₙ-CH₂-CH₂-0-SO₃X, in which R preferably represents a straight-chain or branched, saturated or mono- or polyunsaturated alkyl or alkenyl functional group having 8 to 24 carbon atoms, n represents O or 1 to 12 and X represents an alkali metal or alkaline-earth metal or triethanolamine, anionic surfactants having the INCi name sodium laureth sulfate being particularly preferred, and
aii) at least one amphoteric (zwitterionic) surfactant from the group of (C₈-C₂₄) alkyl ampho(di)acetates and/or propionates and/or from the group of (Cs-C₂₄) alkyl amido(C₁-C₄) alkyl betaines, amphoteric (zwitterionic) surfactants having the INCi names cocoampho(di)acetate and/or cocamidopropyl betaine being particularly preferred.

3. The cosmetic cleaning agent according to one of the preceding claims, characterizedin that the cationic polysaccharide is selected from quaternized cellulose derivatives which may be hydrophobically modified, cationic alkyl polyglycosides, cationized honey and/or cationic guar derivatives, cationic guar derivatives, in particular the guar derivative known by the INCI name guar hydroxypropyltrimonium chloride, being particularly preferred.

4. The cosmetic cleaning agent according to one of the preceding claims, **characterized in that** it contains at least one wax which has a melting point in the range of from 80 to 90 °C, waxes of plant origin being preferred and hydrogenated castor oil being particularly preferred.

5. The cosmetic cleaning agent according to one of the preceding claims, **characterized in that** the cationic protein hydrolyzate is selected from compounds of generalformula (1),
R'-X-R" (1),
in which
- R' represents a straight-chain or branched, saturated or unsaturated hydrocarbon functional group having 8 to 24 carbon atoms,
- R" represents a protein or a protein hydrolyzate,
- X represents -C(O)O- or -N+(R^{III})R^{V}- or -N(R^{III})R^{IV}- or -C(O)-N(R^{V})R^{VI}-,
- R^{III} represents -(CH₂)ₓ-CH₃, where x = 0-22,
- R^{IV} represents -CH₂-CH(OH)-CH₂- or -(CH₂)ₓ-, where x = 0-22, and
- R^{V} and R^{V} represent, independently of one another, -H or -(CH₂)ₓ-CH₃, where x = 0-22, cationic protein hydrolyzates of formula (1) being preferred, in which
- X represents -N⁺ (CH₃)₂-CH₂-CH(OH)-CH₂- or -C(O)-O-,
- R" represents a keratin hydrolysate and
- R' represents -(CH₂)₁₇-CH₃, and
a cationic protein hydrolyzate known by the INCi name cocodimonium hydroxypropyl hydrolyzed keratin being particularly preferred.

6. The cosmetic cleaning agent according to one of the preceding claims,
**characterized in that**
a) the proportion by weight of the anionic and/or amphoteric (zwitterionic) surfactant with respect to the total weight of the composition is in each case 0.5 to 20wt.%, preferably from 1 to 15wt.%,
b) the proportion by weight of the cationic polysaccharide with respect to the total weight of the composition is 0.01 to 5 wt.%, preferably 0.05 to 3 wt.%,
c) the proportion by weight of the silicone with respect to the total weight of the composition is 0.01 to 5 wt.%, preferably 0.05 to 3 wt.%,
d) the proportion by weight of the wax with respect to the total weight of the composition is 0.01 to 2 wt.%, preferably 0.05 to 1wt.%,
e) the proportion by weight of the native oil with respect to the total weight of the composition is 0.01 to 3 wt.%, preferably 0.05 to 2 wt.%, and
f) the proportion by weight of the cationic protein hydrolyzate with respect to the total weight f the composition is 0.01 to 3 wt.%, preferably 0.05 to 2 wt.%.

7. The use of a cosmetic cleaning agent according to one of claims 1 to 6 for eliminating, reducing and/or preventing hair breakage.

8. A method for cleaning hair and/or for eliminating, reducing and/or preventing hair breakage, in which a cosmetic agent containing
a) at least one anionic and/or amphoteric (zwitterionic) surfactant,
b) at least one cationic polysaccharide,
c) at least one silicone that is selected from water-insoluble, non-volatile polydimethyl siloxanes, which have a viscosity at 25 °C in the range of from 30,000 to 100,000 cSt,
d) at least one wax,
e) at least one native oil and
f) at least one cationic protein hydrolyzate
is applied to the preferably wet hair, and is rinsed out using water after a contact time of from 5 seconds to 5 minutes, wherein the weight proportion of the cationic protein hydrolyzate in the total weight of the composition is 0.01 to 3 wt%, wherein the weight proportion of the native oil in the total weight of the composition is 0.01 to 3 wt%.

## Revendications

1. Agent nettoyant cosmétique, contenant dans un support cosmétique
a) au moins un tensioactif anionique et/ou amphotère (zwitterionique),
b) au moins un polysaccharide cationique,
c) au moins une silicone choisie parmi les polydiméthylsiloxanes non hydrosolubles, non volatils, présentant une viscosité a 25°C comprise entre 30 000 et 100 000 cSt,
d) au moins une cire,
e) au moins une huile vierge et
f) au moins un hydrolysat de protéine cationique, la proportion en poids de l'hydrolysat de protéine cationique par rapport au poids total de la composition étant de 0,01 à 3 % en poids, la proportion en poids de l'huile vierge par rapport au poids total de la composition étant de 0,01 à 3 % en poids.

2. Agent nettoyant cosmétique selon la revendication 1, **caractérisé en ce qu'**il
ai) contient au moins un tensioactif anionique choisi dans le groupe constitué des sulfates d'alkyle et/ou des éthersulfates d'alkylpolyglycol de formule R-O-(CH₂-CH₂O)ₙ-CH₂-CH₂-O-SO₃X, dans laquelle R représente de préférence un radical alkyle ou alcenlyle linéaire ou ramifié, saturé ou mono- ou polyinsaturé ayant 8 a 24 atomes de carbone, n représente 0 ou 1 a 12 et X représente un métal alcalin ou alcalinoterreux, ou la triéthanolamine, les tensioactifs anioniques ayant la dénomination INCl sulfates de Laureth de sodium étant particulièrement préférés, et
aii) au moins un tensioactif amphotère (zwitterionique) choisi dans le groupe constitué des (C₈-C₂₄)-alkylampho(di)acétates et/ou des propionates et/ou du groupe constitué des (C₈-C₂₄)-alkylamido(C₁-C₄)-alkylbétaïnes, les tensioactifs amphotère s (zwitterioniques) ayant les dénominations INCl étant de manière particulièrement préférée des cocoampho(di)acétates et/ou de la cocamidopropylbétaïne.

3. Agent nettoyant cosmétique selon l'une des revendications précédentes, **caractérisé en ce que** le polysaccharide cationique est choisi parmi les dérivés de cellulose quaternisés pouvant être hydrophobiquement modifiés, les alkylpolyglycosides cationiques, le miel cationisé et/ou les dérivés de guar cationiques, les dérivés de guar cationiques, en particulier le dérivé de guar connu sous la dénomination INCl chlorures d'hydroxypropyltrimonium de guar, étant particulièrement préférés.

4. Agent nettoyant cosmétique selon l'une des revendications précédentes, **caractérisé en ce qu'**il contient au moins une cire dent le point de fusion est compris entre 80 et 90 °C, les cires d'origine végétale étant préférées et l'huile de ricin durcie étant particulièrement préférée.

5. Agent nettoyant cosmétique selon l'une des revendications précédentes, **caractérisé en ce que** l'hydrolysat de protéine cationique est choisi parmi les composés de formule générale (I),
R'-X-R"
dans laquelle
- R' représente un radical hydrocarboné saturé ou insaturé, linéaire ou ramifié, ayant 8 a 24 atomes de carbone,
- R" signifie une protéine ou un hydrolysat de protéine,
- X représente -C(O)O- or -N⁺(R^{III})R^{V}- ou -N(R^{III})R^{IV}- ou -C(O)-N(R^{V})R^{VI}-,
- R^{III} signifie -(C^{H}₂)ₓ-CH₃ ou x = 0 - - 22,
- R^{IV} signifie CH₂-CH(OH)-CH₂- ou -(CH₂)_{X}- ou x = 0 - 22, et
- R^{V} and R^{V} représentent indépendamment H ou -(C^{H}₂)ₓ-CH₃ ou x = 0-22, les hydrolysats de protéine cationiques selon la formule (I) étant préférés, dans lesquels
- X représente -N⁺ (CH₃)₂-CH₂-CH(OH)-CH₂- ou -C(O)-O-,
- R" représente un hydrolysat de kératine et
- R' représente -(CH₂)₁₇-CH₃, et
un hydrolysat de protéine cationique connu sous la dénomination INCi Kératine hydrolysée d'hydroxypropyl étant particulièrement préféré.

6. Agent nettoyant cosmétique selon l'une des revendications précédentes, **caractérisé en ce que**
a) la proportion en poids du tensioactif anionique et/ou amphotère (zwitterionique) par rapport au poids total de la composition est respectivement comprise entre 0,5 et 20 % en poids, de préférence entre 1 et 15 % en poids,
b) la proportion en poids du polysaccharide cationique par rapport au poids total de la composition est comprise entre 0,01 et 5 % en poids, de préférence entre 0,05 et 3 % en poids,
c) la proportion en poids de silicone par rapport au poids total de la composition est comprise entre 0,01 et 5 % en poids, de préférence entre 0,05 et 3 % en poids,
d) la proportion en poids de la cire par rapport au poids total de la composition est comprise entre 0,01 et 2 % en poids, de préférence entre 0,05 et 1 % en poids,
e) la proportion en poids de l'huile vierge par rapport au poids total de la composition est comprise entre 0,01 et 3 % en poids, de préférence entre 0,05 et 2 % en poids, et
f) la proportion en poids de l'hydrolysat de protéine cationique par rapport au poids total de la composition est comprise entre 0,01 entre 0,05 et 2 % en poids.

7. Utilisation d'un agent nettoyant cosmétique selon l'une des revendications 1à 6 pour éliminer, réduire et/ou éviter la cassure des cheveux.

8. Procédé de nettoyage des cheveux et/ou d'élimination, de réduction et/ou de prévention de la cassure des cheveux, dans lequel une composition cosmétique contenant
a) au moins un tensioactif anionique et/ou amphotère (zwitterionique),
b) au moins un polysaccharide cationique,
c) au moins une silicone choisie parmi les polydiméthylsiloxanes non hydrosolubles, non volatils, présentant une viscosité a 25°C comprise entre 30 000 et 100 000 cSt,
d) au moins une cire,
e) au moins une huile vierge et
f) au moins un hydrolysat de protéine cationique est appliquée sur les cheveux de préférence mouilles et est rincée a l'eau après un temps d'action compris entre 5 secondes et 5 minutes, la proportion en poids de l'hydrolysat de protéine cationique par rapport au poids total de la composition étant de 0,01 à 3 % en poids, la proportion en poids de l'huile vierge par rapport au poids total de la composition étant de 0,01 à 3 % en poids.
